# FASCICULE DE BREVET EUROPEEN

(11) **EP 3 349 059 B1**
(45) Date de publication et mention de la délivrance du brevet: **27.11.2019**
(21) Numéro de dépôt: 18151356.5
(22) Date de dépôt: 12.01.2018
(51) Int. Cl.: G02F 1/1343, B60J 3/04, G02C 7/10, G02F 1/133, G02F 1/137

(54) **ÉCRAN ADAPTATIF SECTORISÉ ET SYSTÈME D'AIDE À LA CONDUITE COMPRENANT UN TEL ÉCRAN ADAPTATIF**
IN SEKTOREN UNTERTEILTER ADAPTIVER ANZEIGEBILDSCHIRM, UND FAHRERASSISTENZSYSTEM, DAS EINEN SOLCHEN ADAPTIVEN BILDSCHIRM UMFASST
SECTORISED ADAPTIVE SCREEN AND DRIVER ASSISTANCE SYSTEM COMPRISING SUCH AN ADAPTIVE SCREEN

(30) Priorité: 17.01.2017 FR 1750332
(43) Date de publication de la demande: 18.07.2018
(73) Titulaire: Valeo Vision, 93012 Bobigny Cedex (FR)
(72) Inventeur: HUE, David, 93012 Cedex BOBIGNY (FR); SATHAYE, Kedar, 93012 Cedex BOBIGNY (FR); DE BOUGRENET, Jean-Louis, 93012 BOBIGNY Cedex (FR); DANIEL, Emmanuel, 93012 BOBIGNY Cedex (FR); DUPONT, Laurent, 93012 BOBIGNY Cedex (FR); ABBAS, Samir, 93012 BOBIGNY Cedex (FR); BENTAHAR, Samir, 93012 BOBIGNY Cedex (FR)
(74) Mandataire: Valeo Vision

(56) Documents cités:
- WO-A1-03/005942
- FR-A1- 2 693 562
- FR-A1- 3 010 941
- US-A1- 2013 215 376
- US-A1- 2014 226 096

## Description

### Domaine technique

La présente invention concerne un écran adaptatif. Elle se situe dans le domaine des dispositifs de protection contre les éblouissements, et plus particulièrement dans le contexte des véhicules automobiles. La présente invention concerne aussi un système d'aide à la conduite comprenant un tel écran adaptatif.

### État de la technique antérieure

On connait des verres statiques, et particulièrement pour des lunettes de soleil, permettant de réduire l'éblouissement du porteur desdites lunettes de soleil. Les verres statiques peuvent être par exemple du type de simples verres teintés, de couleur uniforme ou présentant un gradient de coloration, Ces verres sont dits statiques car ils ne comprennent pas d'éléments dynamiques permettant d'ajuster un coefficient de transmission de la lumière passant au travers desdits verres en fonction d'une luminosité ambiante : la teinte des verres statiques est invariante et ne peut pas être modifiée après leur fabrication. Seule la densité optique des verres est prédéfinie à la fabrication afin de prédéfinir de manière statique et non modifiable le coefficient de transmission desdits verres. Un inconvénient des verres statiques est qu'il n'est pas possible de moduler l'intensité lumineuse transmise par lesdits verres en fonction d'une intensité lumineuse incidente et/ou en fonction d'un certain niveau de seuil de l'intensité lumineuse transmise par les verres statiques.

Pour répondre à ce besoin d'adaptabilité, on connait aussi le document FR 2 975 792 qui divulgue des lunettes comportant des verres dynamiques formés par une couche de cristaux liquides prise entre deux lentilles transparentes. La transmittance des cristaux liquides est modulée en fonction de l'intensité lumineuse mesurée par un photodétecteur située sur les lunettes. Un inconvénient des verres dynamiques est lié au temps de réponse qui est relativement important en fonction de la surface desdits verres dynamiques. Il en résulte que l'obscurcissement des verres actifs connus n'est obtenu qu'après quelques dixièmes de secondes, voire quelques secondes, ce qui les rend inadaptés à certaines situations, notamment dans le contexte de la conduite d'un véhicule automobile par exemple. En effet, dans le cas d'un déplacement par temps ensoleillé au cours duquel le conducteur atteint une région de transition lumineuse rapide et importante, telle que par exemple à l'entrée d'un tunnel, le temps de réponse des verres actifs connus est largement supérieur à la durée de transition lumineuse de ladite région. Par suite, le conducteur du véhicule automobile est généralement contraint d'ôter ses lunettes s'il veut continuer à distinguer correctement la scène de route sur laquelle il se trouve alors.

De manière comparable, en cas de conduite nocturne, les contrastes en intensités lumineuses de la scène de route sont tels - notamment en situation de croisement d'autres véhicules ou lorsque le véhicule pénètre dans une zone éclairée - qu'il est indispensable de minimiser le temps de réponse des verres actifs afin de ne pas rendre leur utilisation complètement inconfortable, voire impossible.

On connait enfin le document FR 6 011 095 qui divulgue un filtre optique adaptatif pour verre de lunettes comprenant au moins deux zones distinctes comprenant des cristaux liquides de manière à adapter son état de transmission optique entre un état opaque et un état transparent. Le problème technique résolu par ce document est de permettre le passage d'un état de transmission optique à un autre de manière progressive. En revanche, ce document ne cherche pas à réduire le temps de réponse des lunettes en fonction d'une variation brutale de l'intensité lumineuse perçue par le porteur des lunettes. Les lunettes divulguées dans ce document ne sont pas compatibles avec une utilisation en conduite nocturne. On connaît également le document WO03/005942 A1 divulguant des lunettes protégeant de l'éblouissement et le document US2013/0215376A1 divulguant des lunettes comportant une partition de zones occultables.

La présente invention a pour objet de répondre au moins en grande partie aux problèmes précédents et de conduire en outre à d'autres avantages.

Un autre but de l'invention est de proposer un nouvel écran adaptatif pour résoudre au moins un de ces problèmes.

Un autre but de la présente invention est de réduire le temps de réponse d'au moins une partie d'un tel écran adaptatif.

Un autre but de la présente invention est de réduire la consommation électrique d'un tel écran adaptatif.

Un autre but de l'invention est d'améliorer le confort d'utilisation d'un tel écran adaptatif.

### Exposé de l'invention

L'invention est définie par les revendications. Les modes de réalisation sont ceux qui comportent au moins toutes les caractéristiques de la revendication 1. Selon un premier aspect de la divulgation, on
atteint au moins l'un des objectifs précités avec un écran adaptatif comprenant au moins un obturateur à cristaux liquides, au moins un des obturateurs comprenant au moins deux zones actives adressées par un signal de commande permettant de faire commuter l'au moins un obturateur correspondant entre une configuration passante dans laquelle une transmittance est égale à une valeur maximale, et une configuration bloquante dans laquelle la transmittance est égale à une valeur minimale, caractérisé en ce qu'une zone active dite principale couvre une surface de l'écran adaptatif inférieure ou égale à 60% de la surface dudit écran adaptatif afin de réduire le temps de réponse de ladite zone active principale, et préférentiellement comprise entre 50% et 60% de la surface de l'écran adaptatif.

L'écran adaptatif conforme au premier aspect permet ainsi de diviser ledit écran adaptatif en une pluralité de zones actives dont chaque transmittance moyenne peut être contrôlée de manière indépendante via le signal de commande correspondant : le ou les obturateurs de la zone active correspondante commute alors entre deux états de transmittance et selon un régime de fonctionnement particulier.

Le régime de fonctionnement des obturateurs est défini par le signal de commande correspondant qui pilote leurs commutations, et plus particulièrement via au moins certaines de ses caractéristiques temporelles comme il sera décrit ultérieurement.

La divulgation conforme à son premier aspect permet ainsi de réduire les surfaces de chaque zone active afin de minimiser le temps de réponse d'au moins la zone active principale. En remplaçant ainsi une unique zone active couvrant toute la surface de l'écran adaptatif par la pluralité de zones actives, il est possible de modifier plus rapidement la transmittance de l'écran adaptatif et/ou la transmittance d'au moins une des zones actives en fonction d'une variation du signal de commande correspondant.

En particulier, l'énergie électrique nécessaire pour faire commuter les obturateurs de chaque zone active d'un état de transmittance à l'autre dépend à la fois de la superficie des zones actives et du régime de fonctionnement appliqué à ladite zone active. Plus spécifiquement, l'énergie électrique nécessaire à la commutation des obturateurs est proportionnelle à la superficie d'une zone active considérée et au régime de fonctionnement auquel elle est soumise. Le régime de fonctionnement des obturateurs conditionne le temps de réponse de la zone active correspondante au travers principalement d'un taux de répétition des transitions entre les deux états de transmittance des obturateurs. Le taux de répétition dépend notamment de la fréquence et/ou de la phase et/ou du rapport cyclique du signal de commande.

Ainsi, la zone active principale de l'écran adaptatif présente une superficie réduite par rapport à celle d'un écran adaptatif équivalent du type de celui de l'art antérieur ; et la superficie de la zone active principale a une superficie inférieure à celle de l'écran adaptatif qui la comprend, de sorte que la consommation d'énergie électrique pour la faire changer d'un état de transmission à un autre et/ou pour faire commuter les obturateurs qui la composent est réduite.

Chaque obturateur est configuré pour commuter - selon un régime de fonctionnement dépendant du signal de commande - entre la configuration bloquante pour laquelle sa transmittance est égale à la valeur minimale, par exemple égale à 0 ou proche de 0, et la configuration passante pour laquelle sa transmittance est égale à la valeur maximale, par exemple égale à 1 ou proche de 1.

La transmittance moyenne de la zone active est ainsi définie par la moyenne temporelle, prise sur une durée d'intégration donnée, des transmittances de chaque obturateur formant ladite zone active. Plus particulièrement, la transmittance moyenne est calculée sur une durée correspondant à quelques cycles de commutation des obturateurs formant ladite zone active, par exemple supérieure à plusieurs centaines de cycles.

On définit aussi une transmittance instantanée lorsque la moyenne temporelle de la transmittance de chaque obturateur formant la zone active est calculée sur une durée correspondant à quelques cycles d'oscillation desdits obturateurs, par exemple comprise entre quelques dizaines à quelques centaines de cycles. En d'autres termes, la transmittance instantanée correspond à la transmittance moyenne calculée sur une durée d'intégration plus faible.

Le terme de transmittance moyenne s'entend ici dans son sens le plus large, et notamment selon l'une des deux interprétations évoquées ci-dessus.

Il est ainsi possible de piloter chaque obturateur de l'écran adaptatif selon un régime différent afin de contrôler la configuration correspondante de la ou des zones actives formées par chaque obturateur. À titre d'exemple non limitatif :
- la transmittance moyenne d'une première zone active pourrait être configurée de manière à être proche de 0, la zone active étant complètement ou quasiment complètement occultée, et/ou
- la transmittance moyenne d'une deuxième zone active pourrait être configurée de manière à être proche de 1, la zone active étant complètement ou quasiment complètement transparente, et/ou
- la transmittance moyenne d'une troisième zone active pourrait être configurée de manière à être égale à une valeur intermédiaire, par exemple proche de 0.5.

Une transmittance moyenne tendant vers 0 permet d'atténuer le flux de lumière traversant la zone active correspondante, elle permet par exemple d'éviter un éblouissement ou une gêne lorsque le flux de lumière parvenant à l'écran est très important. A contrario, une transmittance moyenne tendant vers 1 permet de laisser passer le flux de lumière traversant la zone active correspondante en limitant son atténuation, voire sans l'atténuer. Une telle valeur de transmittance moyenne est particulièrement adaptée à une situation de faible luminosité dans laquelle par exemple l'intensité du flux de lumière parvenant l'écran est suffisamment faible pour ne pas provoquer un éblouissement ou une gêne.

Selon d'autres modes de réalisation, le nombre, la surface et la forme des zones actives ainsi que la variété des régimes de fonctionnement peut être quelconque en fonction des applications et des besoins recherchés. En particulier, certaines zones actives peuvent être commandées par un signal de commande identique. De manière très particulière, la présente invention propose notamment un écran actif comprenant une pluralité de zones actives qui sont toutes pilotées par un même signal de commande, ou par une pluralité de signaux de commande identiques. Un tel écran adaptatif, bien que proposant en apparence une seule surface de transmittance variable, aurait cependant un temps de réponse plus court que les écrans adaptatifs connus du fait de sa division en plusieurs zones actives.

L'écran adaptatif conforme au premier aspect de la divulgation peut comprendre avantageusement au moins un des perfectionnements ci-dessous, les caractéristiques techniques formant ses perfectionnements pouvant être prises seules ou en combinaison :
- l'au moins un obturateur est logé entre un premier et/ou un deuxième support transparent. Le premier et/ou le deuxième support transparent peut former une lentille de manière à modifier la trajectoire d'au moins un rayon lumineux traversant respectivement le premier et/ou le deuxième support transparent. La ou les lentilles ainsi formées peuvent permettre par exemple de corriger un problème ophtalmologique connu, tel que la myopie ou l'astigmatisme ;
- l'écran adaptatif comprend au moins trois électrodes, dont une première partie est située d'un premier côté de l'au moins un obturateur et une deuxième partie est située d'un deuxième côté de l'au moins un obturateur, les électrodes permettant d'appliquer un champ électrique non nul entre elles pour configurer l'au moins un obturateur de chaque zone active dans la configuration passante ou bloquante, chaque zone active pouvant être configurée indépendamment des autres zones actives. De manière plus particulière, une zone active formée par au moins un obturateur à cristaux liquides peut comporter un premier filtre polarisant, dit polariseur, et/ou un deuxième filtre polarisant, dit analyseur, et au moins une couche de cristaux liquides située entre le polariseur et l'analyseur. Les électrodes sont fixées sur les faces des filtres polarisant situées en regard des cristaux liquides. L'application du champ électrique entre deux électrodes opposées par rapport à l'obturateur correspondant permet de polariser électriquement la couche de cristaux liquides selon un état de polarisation optique particulier. À chaque état de polarisation électrique correspond un état de polarisation optique et, in fine, un état de transmittance moyenne de la zone active correspondante. Ainsi, l'application d'un champ électrique variable - et plus particulièrement périodique - permet de modifier l'état de polarisation optique des obturateurs de la zone active correspondante - et plus particulièrement de manière périodique - et donc de modifier l'état de transmittance de la zone active correspondante. Chaque électrode est avantageusement située de part et d'autre des obturateurs, par exemple contre chaque face des support transparents situées en regard desdits obturateurs. Alternativement, l'écran adaptatif comprend au moins trois électrodes dont une première partie est située d'un premier côté de l'au moins un obturateur et une deuxième partie est située d'un deuxième côté de l'au moins un obturateur, les électrodes permettant d'appliquer un champ électrique non nul entre elles pour configurer l'au moins un obturateur de chaque zone active dans la configuration passante ou bloquante, chaque zone active pouvant être configurée indépendamment des autres zones actives. De manière plus particulière, une zone active formée par au moins un obturateur peut comprendre un dopant dichroïque et/ou chiral. Les électrodes sont fixées sur des faces opposées et en regard par rapport aux cristaux liquides. L'application du champ électrique entre deux électrodes opposées par rapport à l'obturateur correspondant permet de faire basculer les molécules formant le dopant dichroïque et/ou chiral entre un premier état dans lequel elles réfléchissent un rayon lumineux incident et un deuxième état dans lequel elles absorbent un rayon lumineux incident. In fine, un état de transmittance moyenne de la zone active correspondante peut être obtenu en fonction d'un rapport cyclique entre les états absorbant et réfléchissant desdites molécules de colorant dichroïque. Chaque électrode est avantageusement située de part et d'autre des obturateurs, par exemple contre chaque face des support transparents situées en regard desdits obturateurs ;
- l'écran adaptatif comprend des conducteurs électriques pour conduire le signal de commande vers au moins une partie des électrodes. Plus particulièrement, un conducteur électrique est relié électriquement à la première électrode, un deuxième conducteur électrique est relié à la deuxième électrode, et un troisième conducteur électrique est relié à la troisième électrode ;
- les conducteurs électriques sont connectés électriquement à chacune des électrodes au niveau d'au moins une interface de connexion électrique, chaque interface de connexion électrique s'étendant le long d'une zone active ;
- l'interface de connexion électrique s'étend de manière périphérique à au moins une zone active, et plus particulièrement au niveau d'au moins une partie des zones inactives ;
- l'interface de connexion électrique s'étend de manière périphérique à l'écran adaptatif de manière à ne pas être visible au travers dudit écran adaptatif ;
- au moins une interface de connexion électrique s'étend le long de la zone active correspondante sur une distance comprise entre 0,1 cm et 2,5 cm. Avantageusement, chaque zone active comprend deux interfaces de connexion électrique de longueur sensiblement égale à 4 mm et distantes de au moins un millimètre ;
- l'au moins un obturateur est configuré en fonction d'au moins un paramètre du signal de commande, le paramètre étant choisi parmi une fréquence de commutation et/ou une phase de commutation et/ou un rapport cyclique de commutation. Ainsi, les paramètres temporels du signal de commande permettent d'appliquer un champ électrique variable sur au moins une partie des zones actives, et plus particulièrement sur les obturateurs formant lesdites zones actives. Comme décrit précédemment, l'application d'un tel champ électrique permet de modifier l'état de polarisation optique - dans le cas où l'obturateur est associé à au moins un filtre polarisant - ou l'état d'absorption et/ou de réflexion optique - dans le cas où l'obturateur est associé à un dopant dichroïque et/ou chiral. La modification de ces états permet de faire commuter l'obturateur entre les différents états correspondant de transmittance, tels que définis précédemment. Préférentiellement, le signal de commande est périodique et permet donc de contrôler l'état de transmittance de la zone active correspondante ;
- le signal de commande est du type d'un signal à largeur d'impulsion modulée, dit signal de commande PWM (pour l'expression anglophone « *Pulse Width Modulation* »). D'une manière plus générale, le signal de commande est du type d'un signal électrique périodique, préférentiellement numérique, dont la fréquence d'oscillation est comprise entre 100 Hz et 1 kHz. L'utilisation d'un signal périodique permet avantageusement d'augmenter le taux de répétition des transitions entre les deux états de polarisation et/ou de transmittance de l'au moins un obturateur, et donc de minimiser l'énergie électrique nécessaire au fonctionnement de la zone active correspondante et, plus généralement, de l'écran adaptatif. Bien sûr, en fonction des dimensions de l'écran adaptatif, les temps de réponses des différentes zones actives peuvent varier. Cependant, d'une manière générale, l'écran adaptatif conforme au premier aspect de la divulgation permet de réduire le temps de réponse d'au moins une zone active par rapport à un écran adaptatif de taille identique ne comprenant qu'une seule zone active ;
- la fréquence de commutation de l'au moins un obturateur de la zone active principale est supérieure ou égale à 200 Hz de manière à rendre imperceptibles à l'œil humain les commutations dudit au moins un obturateur de la zone active principale. Éventuellement, la fréquence de commutation de l'au moins un obturateur de la zone active principale est supérieure à celle d'au moins une autre zone active de l'écran adaptatif. Ainsi, pour une énergie électrique donnée, cette caractéristique avantageuse permet d'augmenter le taux de répétition de la zone active principale et de prioritairement optimiser le fonctionnement de ladite zone principale par rapport aux autres zones actives de l'écran adaptatif, notamment en minimisant son temps de réponse ;
- chaque zone active est délimitée et/ou séparée d'une autre zone active par une zone inactive. Une zone active est définie comme étant une zone de l'écran adaptatif qui ne comprend pas d'obturateurs ou alternativement comme étant une zone dont l'au moins un obturateur n'est pas adressé électriquement, de sorte qu'aucun champ électrique ne peut être appliqué à leurs bornes afin d'en modifier l'état de polarisation et/ou de transmittance. Préférentiellement, au moins les zones actives qui sont situées dans des zones centrales de l'écran adaptatif sont transparentes. Préférentiellement, les zones inactives comprennent au moins une partie des interfaces de connexion électrique ;
- la largeur de la zone inactive est inférieure à 2 µm de manière à les rendre imperceptibles à l'œil humain ;
- les zones actives sont adjacentes deux à deux, chaque zone active s'étendant d'un bord à l'autre de l'écran adaptatif. Selon un premier mode de réalisation, les zones actives sont organisées en bandes horizontales, les zones actives étant adjacentes deux à deux dans la direction verticale, chaque zone active étant séparée de la zone active adjacente par une zone inactive. Selon un deuxième mode de réalisation, les zones actives sont organisées en bandes verticales, les zones actives étant adjacentes deux à deux dans la direction horizontale, chaque zone active étant séparée de la zone active adjacente par une zone inactive. Selon d'autres modes de réalisation, les zones actives peuvent s'étendre dans une direction quelconque suivant les applications considérées. Éventuellement, les zones actives peuvent former des zones géométriques variées afin par exemple de correspondre à certaines zones ophtalmologiques particulières, comme par exemple une première zone adaptée à la vision de près et une deuxième zone adaptée à une vision de loin. D'autres configurations seront décrites ultérieurement à titre d'exemples.

Selon un deuxième aspect de la divulgation, il est proposé un équipement de protection optique comprenant un écran adaptatif conforme au premier aspect de la divulgation ou à l'un quelconque de ses perfectionnements et un support agencé pour maintenir l'écran adaptatif devant les yeux d'un individu portant ledit équipement de protection optique, la zone active principale de l'écran adaptatif étant située dans une zone centrale de l'écran adaptatif et s'étendant latéralement d'un bord à l'autre de l'écran adaptatif, la surface de ladite zone de conduite étant inférieure ou égale à 60% de la surface de l'écran adaptatif.

La divulgation conforme à son deuxième aspect permet ainsi d'améliorer le confort visuel de l'individu qui porte l'équipement de protection optique, notamment du point de vue de la luminosité perçue par ledit individu. Plus particulièrement, il est possible de configurer chaque zone active de l'écran adaptatif de l'équipement de protection optique conforme au deuxième aspect en fonction de la luminosité ambiante, et plus particulièrement en fonction de la luminosité d'une scène perçue par l'individu au travers dudit écran adaptatif.

De manière encore plus spécifique, la divulgation conforme à son deuxième aspect permet avantageusement de configurer la transmittance de chaque zone active en fonction de la partie de la scène de vue qui est située dans l'axe de chaque zone active correspondante par rapport aux yeux de l'individu. La transmittance de chaque zone active est alors configurée de manière à rendre conforme une luminance dite transmise par rapport à une valeur prédéfinie de luminance.

Pour une source lumineuse donnée, on définit la luminance transmise par la luminance perçue par l'individu derrière l'écran adaptatif, en comparaison à une luminance dite incidente correspondant à la luminance de ladite source lumineuse.

Pour rappel, en photométrie, la luminance lumineuse ou luminance visuelle, couramment nommée luminance, se définit comme le quotient de l'intensité lumineuse de la surface source par l'aire de cette source projetée sur un plan perpendiculaire à la direction d'observation. L'unité S1 de la luminance est la candela par mètre carré (cd·m⁻²) ; et elle dépend de la sensibilité de l'œil humain.

Un tel équipement de protection optique peut par exemple être configuré pour que la luminance transmise soit homogène ou sensiblement homogène sur une partie de la surface de l'écran adaptatif. En d'autres termes, l'équipement de protection optique peut être configuré pour qu'une même quantité de rayons lumineux traversent au moins une partie des zones actives par unité de temps. À tout le moins, l'équipement de protection optique conforme au deuxième aspect est agencé pour minimiser les écarts de luminance transmise entre chaque zone active de l'écran adaptatif.

À titre d'exemple non limitatif, on considère un individu portant un tel équipement de protection et regardant une scène à fort contraste lumineux, composée d'une région sombre et d'une région lumineuse. Une première zone active de l'écran adaptatif alignée avec les yeux de l'individu d'une part et la région sombre d'autre part peut être configurée pour que sa transmittance soit égale ou proche de la valeur maximale - typiquement égale à 1 - afin d'être transparente et de laisser passer une majorité des rayons lumineux arrivant sur ladite première zone active. A contrario, une deuxième zone active de l'écran adaptatif alignée avec les yeux de l'individu d'une part et la région lumineuse d'autre part peut être configurée pour que sa transmittance soit égale ou proche de la valeur minimale - typiquement égale à 0 - afin d'être plus opaque que la première zone active et d'atténuer une grande partie des rayons lumineux arrivant sur ladite deuxième zone active.

Les zones actives de l'écran adaptatif d'un tel équipement de protection optique peuvent former des zones géométriques variées en fonction des considérations. À titre d'exemples non limitatifs, les zones actives d'un tel écran adaptatif peuvent être adjacentes deux à deux, chaque zone active s'étendant d'un bord à l'autre de l'écran adaptatif. Selon un premier mode de réalisation, les zones actives peuvent être organisées en bandes horizontales, les zones actives étant adjacentes deux à deux dans la direction verticale, chaque zone active étant séparée de la zone active adjacente par une zone inactive. Selon un deuxième mode de réalisation, les zones actives peuvent être organisées en bandes verticales, les zones actives étant adjacentes deux à deux dans la direction horizontale, chaque zone active étant séparée de la zone active adjacente par une zone inactive. Selon d'autres modes de réalisation, les zones actives peuvent s'étendre dans une direction quelconque suivant les applications considérées. Éventuellement, les zones actives peuvent être confondues avec certaines zones ophtalmologiques particulières d'une paire de lunette ou d'une visière de casque de moto, afin par exemple de correspondre à une première zone adaptée à la vision de près et une deuxième zone adaptée à une vision de loin. D'autres configurations seront décrites ultérieurement à titre d'exemples.

En particulier, les exemples et caractéristiques techniques relatives aux zones actives des écrans adaptatifs décrits dans les premiers, troisièmes et quatrièmes aspects de la divulgation s'appliquent *mutatis mutandis* à l'écran adaptatif de l'équipement de protection optique.

L'équipement de protection optique conforme au deuxième aspect peut comprendre avantageusement au moins un des perfectionnements ci-dessous, les caractéristiques techniques formant ses perfectionnements pouvant être prises seules ou en combinaison :
- l'équipement de protection optique est du type d'un casque de moto dans lequel le support forme au moins en partie une coque dudit casque de moto et une visière dudit casque de moto est formée par au moins une partie de l'écran adaptatif. Bien sûr, en fonction des dimensions de l'écran adaptatif, les temps de réponses des différentes zones actives peuvent varier. Cependant, d'une manière générale, l'écran adaptatif conforme au premier aspect permet de réduire le temps de réponse d'au moins une zone active par rapport à un écran adaptatif de taille identique ne comprenant qu'une seule zone active ;
- l'équipement de protection optique est du type d'une paire de lunettes dans laquelle le support forme une monture de ladite paire de lunettes et au moins une partie d'un verre de la paire de lunettes est formé par l'écran adaptatif ;
- l'équipement de protection optique comprend un dispositif de stockage d'énergie électrique relié électriquement à au moins un conducteur électrique pour polariser électriquement l'au moins un obturateurs d'au moins une zone active. De préférence, le dispositif de stockage d'énergie est logé dans le support de l'équipement de protection optique, par exemple dans la monture de la paire de lunettes ou dans la coque du casque. Le dispositif de stockage d'énergie peut prendre la forme de n'importe quelle batterie électrique connue ;
- l'au moins un obturateur comprend une pluralité de cristaux liquides et au moins un polariseur permettant de configurer l'obturateur correspondant entre la configuration passante et la configuration bloquée ;
- l'au moins un obturateur comprend un mélange à cristal liquide et au moins un polariseur permettant de configurer l'obturateur correspondant entre la configuration passante et la configuration bloquée ;
- l'au moins un obturateur comprend un mélange à cristal liquide avec au moins un dopant dichroïque et/ou chiral pouvant être configuré entre la configuration passante et la configuration bloquée ;
- l'au moins un obturateur comprend une pluralité de cristaux liquides avec au moins un dopant dichroïque et/ou chiral pouvant être configuré entre la configuration passante et la configuration bloquée. Le dopant dichroïque et/ou chiral présente des propriétés d'absorption et/ou de réflexion lorsqu'il est utilisé en combinaison avec des cristaux liquides, et notamment lorsque la concentration en dopant dichroïque et/ou chiral est suffisante. Dans ce mode de réalisation particulier de l'invention, les dopants présents peuvent s'orienter dans la direction des directeurs des molécules cristal liquide ;
- l'équipement de protection optique comprend un dispositif de stockage d'énergie électrique relié électriquement à au moins un des conducteurs électriques pour modifier la configuration du dopant dichroïque d'au moins une zone active ;
- l'équipement de protection optique comprend une unité de contrôle configurée pour générer le signal de commande de manière à faire commuter l'au moins un obturateur d'au moins une zone active entre la configuration passante et la configuration bloquée. L'unité de contrôle est électriquement reliée auxdites zones actives par l'intermédiaire de fils de connexion électrique. De préférence, l'unité de contrôle est logée dans le support de l'équipement de protection optique, par exemple dans la monture de la paire de lunettes ou dans la coque du casque ;
- l'unité de contrôle génère au moins un signal de commande par zone active afin de configurer une transmittance moyenne prédéterminée pour chaque zone active ;
- l'équipement de protection optique comprend en outre un photodétecteur agencé pour mesuré au moins une intensité lumineuse, l'au moins un signal de commande étant défini en fonction de l'intensité lumineuse mesurée. Le photodétecteur peut prendre par exemple la forme d'une caméra, par exemple du type CMOS (pour l'expression anglophone « *Complementary Metal-Oxyde Semiconductor* », semi-conducteur d'oxyde de métal complémentaire) ou CCD (pour l'expression anglophone « *Charge Coupled Device* », dispositif à transfert de charges). De préférence, le photodétecteur est logé dans le support de l'équipement de protection optique, par exemple dans la monture de la paire de lunettes ou dans la coque du casque. Préférentiellement, il est situé à proximité de l'écran adaptatif, entre l'individu portant l'équipement de protection optique et l'écran adaptatif, ou alternativement du côté opposé de l'écran adaptatif par rapport audit individu.

Selon un troisième aspect de la divulgation, il est proposé un système d'aide à la conduite d'un véhicule automobile comprenant :
- un écran adaptatif conforme au premier aspect ou à l'un quelconque de ses perfectionnements ;
- une unité de contrôle d'au moins une partie des zones actives de l'écran adaptatif, l'unité de contrôle étant configurée pour générer au moins un signal de commande de l'au moins un obturateur de l'écran adaptatif.

La divulgation conforme à son troisième aspect permet ainsi d'améliorer le confort visuel d'un conducteur de véhicule automobile, notamment du point de vue de la luminosité perçue par ledit conducteur. Plus particulièrement, l'unité de contrôle permet de configurer chaque zone active de l'écran adaptatif en fonction au moins de la luminosité d'une scène de route située devant le véhicule automobile et perçue par le conducteur au travers dudit écran adaptatif.

D'une manière générale, le terme véhicule automobile est ici pris dans son sens le plus large, en tant que véhicule automobile roulant, notamment motorisé. À titre d'exemples non limitatif, il peut s'agir d'une voiture, d'une moto, d'un camion ou encore d'un car.

L'unité de contrôle peut rendre la forme d'un microcontrôleur ou d'un microprocesseur.

De manière comparable aux premiers et deuxièmes aspects, la divulgation conforme à son troisième aspect permet avantageusement de configurer la transmittance de chaque zone active en fonction de la partie de la scène de route qui est située dans l'axe de chaque zone active correspondante par rapport aux yeux du conducteur. La transmittance de chaque zone active est alors configurée de manière à rendre conforme une luminance transmise par rapport à une valeur prédéfinie afin d'améliorer le confort visuel du conducteur.

L'unité de contrôle d'un tel système d'aide à la conduite peut par exemple être configurée pour que la luminance transmise soit homogène ou sensiblement homogène sur tout ou partie de la surface de l'écran adaptatif, c'est-à-dire sur tout ou partie des zones actives. À tout le moins, le système d'aide à la conduite conforme au troisième aspect est agencé pour minimiser les écarts de luminance transmise entre chaque zone active de l'écran adaptatif.

À titre d'exemple non limitatif, on considère un conducteur portant un tel équipement de protection et regardant une scène de route en plein soleil. Ce type de scène de route peut présenter de forts contrastes lumineux, la partie située au-dessus de l'horizon pouvant être très lumineuse, surtout si le soleil y est directement visible, tandis que la partie médiane correspondant à la partie de la route située devant le véhicule automobile pouvant présenter une luminosité beaucoup moins importante en comparaison avec la partie supérieure. Ainsi, une première zone active de l'écran adaptatif alignée avec les yeux du conducteur d'une part et la partie de la scène de route correspondant à la route située devant le véhicule automobile peut être configurée par l'unité de contrôle de manière à ce que sa transmittance soit égale ou proche de la valeur maximale - typiquement égale à 1 - afin d'être transparente et de laisser passer une majorité du flux lumineux arrivant sur ladite première zone active. A contrario, une deuxième zone active de l'écran adaptatif alignée avec les yeux du conducteur d'une part et la partie de la scène de route située au-dessus de l'horizon d'autre part peut être configurée par l'unité de contrôle pour que sa transmittance soit égale ou proche de la valeur minimale - typiquement égale à 0 - afin d'être plus opaque que la première zone active et d'atténuer le flux lumineux arrivant sur ladite deuxième zone active.

Les zones actives de l'écran adaptatif d'un tel système d'aide à la conduite peuvent former des zones géométriques variées en fonction des considérations. À titre d'exemples non limitatifs, les zones actives d'un tel écran adaptatif peuvent être adjacentes deux à deux, chaque zone active s'étendant d'un bord à l'autre de l'écran adaptatif. Selon un premier mode de réalisation, les zones actives peuvent être organisées en bandes horizontales, les zones actives étant adjacentes deux à deux dans la direction verticale, chaque zone active étant séparée de la zone active adjacente par une zone inactive. Selon un deuxième mode de réalisation, les zones actives peuvent être organisées en bandes verticales, les zones actives étant adjacentes deux à deux dans la direction horizontale, chaque zone active étant séparée de la zone active adjacente par une zone inactive. Selon d'autres modes de réalisation, les zones actives peuvent s'étendre dans une direction quelconque suivant les applications considérées. Éventuellement, les zones actives peuvent être confondues avec certaines zones ophtalmologiques particulières d'une paire de lunette ou d'une visière de casque de moto, afin par exemple de correspondre à une première zone adaptée à la vision de près et une deuxième zone adaptée à une vision de loin. D'autres configurations seront décrites ultérieurement à titre d'exemples.

Le système d'aide à la conduite conforme au troisième aspect peut comprendre avantageusement au moins un des perfectionnements ci-dessous, les caractéristiques techniques formant ses perfectionnements pouvant être prises seules ou en combinaison :
- l'unité de contrôle est configurée pour faire commuter l'au moins un obturateur de la zone active correspondante entre la configuration passante et la configuration bloquée, chaque zone active étant contrôlée par un signal de commande égal ou différent du signal de commande des autres zones actives. De cette manière, chaque zone active de l'écran adaptatif est contrôlée indépendamment des autres zones actives afin d'ajuster sa transmittance en fonction de la partie de la scène de route à laquelle elle est associée, c'est-à-dire préférentiellement la partie de la scène de route qui est sensiblement alignée avec - ou au voisinage de - l'œil du conducteur du véhicule automobile. Pour ce faire, au moins un paramètre de commande du signal de commande associé à au moins une zone active est différent du paramètre de commande associé à une autre zone active. À titre d'exemple non limitatif, une première zone active peut être configurée avec un premier rapport cyclique, une deuxième zone active peut être configurée avec un deuxième rapport cyclique différent du premier rapport cyclique, et une troisième zone active peut être configurée via un signal de commande ayant une fréquence de commutation différente de celle des première et deuxième zones actives ;
- le système d'aide à la conduite comprend en outre (i) un photodétecteur agencé pour détecter au moins un rayon lumineux atteignant le véhicule automobile depuis une scène de route située devant ledit véhicule automobile ; et (ii) une unité de traitement configurée pour déterminer une intensité lumineuse du rayon lumineux, l'au moins un signal de commande généré par l'unité de contrôle étant déterminé en fonction de l'intensité lumineuse. Un tel système d'aide à la conduite permet ainsi de configurer chaque zone active en fonction du rayon lumineux atteignant le véhicule automobile et de mesurer l'intensité lumineuse associée. Par la suite, en comparant par exemple l'intensité lumineuse du rayon atteignant le véhicule automobile par rapport à une valeur seuil correspondant à une valeur d'intensité lumineuse confortable pour le conducteur, alors l'unité de traitement transmet à l'unité de contrôle un paramètre qui permet de déterminer dans quelle mesure le flux lumineux des rayons lumineux atteignant le véhicule automobile doit être absorbé par l'écran adaptatif afin de ne transmettre au conducteur qu'une partie des rayons lumineux atteignant le véhicule automobile. Bien entendu, ce traitement est réalisé pour chaque zone active, de manière indépendante. Chaque zone active peut être associée à une valeur seuil particulière. Les valeurs seuils peuvent aussi évoluer dans le temps, en fonction de l'utilisation et de la situation du véhicule automobile. Par exemple, des premières valeurs seuil peuvent être définies pour un usage diurne du véhicule automobile, et des deuxièmes valeurs seuil peuvent être définies pour un usage nocturne ;
- le photodétecteur est situé entre l'écran adaptatif et un conducteur du véhicule automobile, le rayon lumineux traversant d'abord l'écran adaptatif avant d'atteindre ledit photodétecteur. Cette configuration astucieuse permet d'améliorer la précision des mesures réalisées et d'améliorer le confort obtenu via un tel système d'aide à la conduite. En effet, dans ce cas, le photodétecteur est agencé pour mesurer une intensité lumineuse tel qu'elle est perçue par l'œil ; et la correction réalisée conjointement par l'unité de traitement et l'unité de contrôle pour configurer les zones actives de l'écran afin d'atténuer une partie de l'intensité lumineuse atteignant l'écran adaptatif est ainsi plus précise que lorsque le photodétecteur est situé dans une configuration plus lointaine par rapport à l'œil, et notamment de l'autre côté de l'écran adaptatif par rapport à l'œil ;
- au moins une zone active de l'écran adaptatif est configurée pour réduire un scintillement et/ou un éblouissement véhicule automobile détecté par le photodétecteur, notamment en réduisant l'intensité lumineuse d'une partie des rayons lumineux traversant l'écran adaptatif ;
- l'écran adaptatif est formé par au moins une partie du pare-brise du véhicule automobile, ou un écran disposé entre le pare-brise et un conducteur du véhicule automobile, ou au moins un verre d'une paire de lunettes portée par le conducteur du véhicule automobile, ou au moins une partie d'une visière d'un casque porté par le conducteur du véhicule automobile ;
- lorsque l'écran adaptatif est formé par la paire de lunettes ou le casque, l'écran adaptatif comprend au moins une zone active dite de pare-soleil et située sur une partie supérieure de l'écran adaptatif et s'étendant latéralement d'un bord à l'autre de l'écran adaptatif. Typiquement, la zone de pare-soleil couvre une partie de la scène de route située au-dessus de l'horizon. La zone de pare-soleil est formée par un bandeau sensiblement horizontal couvrant la partie supérieure de l'écran adaptatif. Éventuellement, la zone de pare-soleil est légèrement incurvée en fonction de la forme de l'écran adaptatif, la zone de pare-soleil formant principalement une bande de largeur constante d'un bord latéral à l'autre de l'écran adaptatif ;
- lorsque l'écran adaptatif est formé par la paire de lunettes ou le casque, l'écran adaptatif comprend une zone active dite de vision de près située dans un coin inférieur de l'écran adaptatif, la surface de ladite zone de vision de près étant supérieure ou égale au quart de la surface de l'écran adaptatif. Typiquement, la zone de vision de près couvre une partie du champ visuel correspondant à une partie du tableau de bord du véhicule automobile. La zone de vision de près est typiquement formée par une surface formant un secteur d'un disque situé sur la partie inférieure et centrale de l'écran adaptatif. Plus particulièrement, dans le cas d'une paire de lunettes, la zone de vision de près est formée par un secteur de disque situé dans le coin inférieur et central de chaque verre de lunette, le secteur couvrant sensiblement une surface d'un quart de cercle. Dans le cas d'une visière de casque de moto, la zone de vision de près est formée par un secteur de disque situé latéralement au centre et verticalement vers le bas de ladite visière, le secteur couvrant sensiblement une surface d'un demi-cercle. Éventuellement, la zone de vision de près peut couvrir un bandeau s'étendant latéralement d'un bord à l'autre de l'écran adaptatif et de largeur constante ;
- l'au moins un obturateur de la zone de vision de près est configuré en opposition de phase avec un éclairage intérieur du véhicule automobile afin de pouvoir atténuer le contraste d'intensité lumineuse entre les faisceaux émis par un dispositif d'éclairage du véhicule automobile et l'éclairage intérieur dudit véhicule automobile. Cette caractéristique est particulièrement avantageuse pour un trajet nocturne afin d'améliorer le confort et de réduire la fatigue visuelle du conducteur ;
- lorsque l'écran adaptatif est formé par la paire de lunettes ou le casque, l'écran adaptatif comprend une zone active dite de conduite et située dans une zone centrale de l'écran adaptatif et s'étendant latéralement d'un bord à l'autre de l'écran adaptatif, la surface de ladite zone de conduite étant supérieure ou égale à la moitié de la surface de l'écran adaptatif. Typiquement, la zone de conduite couvre une partie du champ visuel correspondant à la scène de route située devant le véhicule automobile, et notamment la route. La zone de conduite est typiquement formée par une surface incurvée s'étendant latéralement d'un bord à l'autre de l'écran adaptatif et située par exemple entre la zone de vision de près et la zone de pare-soleil. La zone de conduite peut être plus étroite sur une zone centrale de l'écran adaptatif, et plus large sur les bords de l'écran adaptatif. Alternativement, la zone de conduite peut prendre la forme d'un bandeau d'épaisseur constante et s'étendant latéralement d'un bord à l'autre de l'écran adaptatif ;
- la zone de conduite est agencée pour couvrir une zone perçue par le conducteur couvrant au moins en partie la scène de route.
- le système d'aide à la conduite comprend un oculomètre agencé pour mesurer un mouvement des yeux du conducteur du véhicule automobile et/ou un dispositif de suivi d'un mouvement de la tête du conducteur du véhicule automobile, au moins une zone active de l'écran adaptatif étant configurée en fonction dudit mouvement des yeux et/ou dudit mouvement de la tête respectivement.

Selon un quatrième aspect de la divulgation, il est proposé un véhicule automobile comprenant (i) au moins un dispositif d'éclairage agencé pour émettre un faisceau d'éclairage de la route située en avant du véhicule automobile, (ii) un système d'aide à la conduite conforme au deuxième aspect ou selon n'importe lequel de ses perfectionnements, ledit système d'aide à la conduite étant agencé pour déterminer l'au moins un paramètre du signal de commande de l'écran adaptatif et pour piloter l'allumage d'au moins une source lumineuse du dispositif d'éclairage en fonction dudit au moins un paramètre.

Le véhicule automobile conforme au quatrième aspect peut comprendre avantageusement au moins un des perfectionnements ci-dessous, les caractéristiques techniques formant ses perfectionnements pouvant être prises seules ou en combinaison :
- l'au moins un obturateur d'au moins une zone active de l'écran adaptatif est synchronisé avec l'au moins une source lumineuse du dispositif d'éclairage. Plus particulièrement, la fréquence de commutation et/ou la phase de commutation du signal de commande de l'au moins un obturateur d'au moins une zone active de l'écran adaptatif est synchronisée avec l'au moins une source lumineuse. En d'autres termes, une unité de contrôle contrôlant l'émission du faisceau de lumière émis par les sources lumineuses du dispositif d'éclairage est configurée pour émettre un signal de synchronisation à l'unité de contrôle du dispositif d'aide à la conduite afin de synchroniser au moins un zone active, par exemple en réglant la fréquence de commutation du signal de commande d'au moins une zone active à la même fréquence qu'une fréquence de pulsation du dispositif d'éclairage. Cette caractéristique avantageuse permet notamment de pouvoir régler un contraste lumineux entre au moins une zone active et le dispositif d'éclairage dudit véhicule automobile ;
- l'au moins un obturateur d'au moins une zone active de l'écran adaptatif commute en phase avec l'au moins une source lumineuse du dispositif d'éclairage.

Des modes de réalisation variés de l'invention sont prévus, intégrant selon l'ensemble des caractéristiques de la revendication 1.

### Description des figures

D'autres caractéristiques et avantages de l'invention apparaîtront encore au travers de la description qui suit d'une part, et de plusieurs exemples de réalisation donnés à titre indicatif et non limitatif en référence aux dessins schématiques annexés d'autre part, sur lesquels :
- les FIGURES 1a et 1b illustrent deux obturateurs d'un écran adaptatif conforme au premier aspect de la divulgation ;
- les FIGURES 2a et 2b illustrent deux variantes de réalisation d'un équipement de protection optique prenant la forme d'une paire de lunettes conforme au deuxième aspect de la divulgation ;
- les FIGURES 3a et 3b illustrent deux variantes de réalisation d'un équipement de protection optique prenant la forme d'un casque de moto conforme au deuxième aspect de la divulgation ;
- la FIGURE 4 illustre une vue schématique d'un système d'aide à la conduite conforme au troisième aspect de la divulgation.

Sur les figures, les éléments communs à plusieurs figures conservent la même référence.

### Description détaillée de l'invention

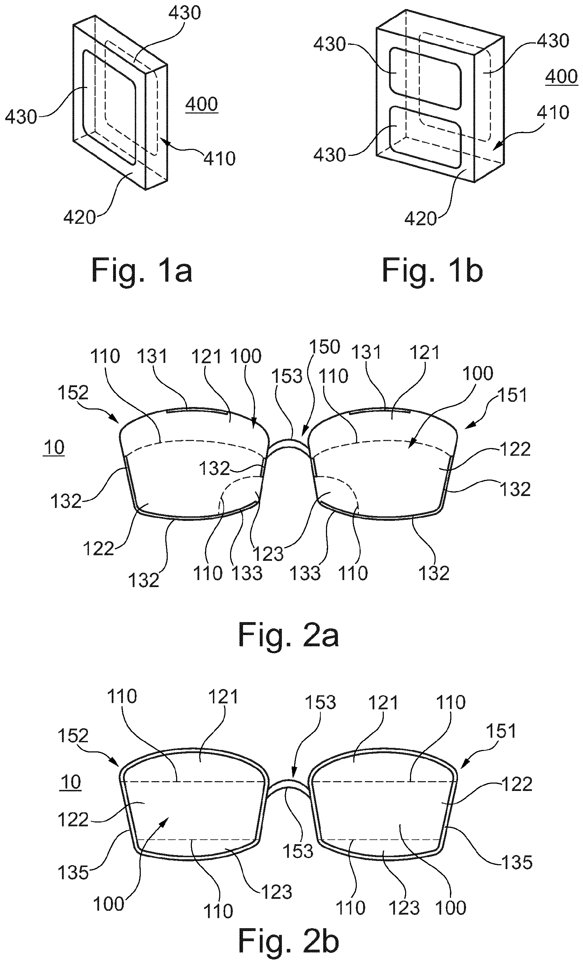

En référence aux FIGURES 1a et 1b un obturateur tel que mis en œuvre dans un écran adaptatif conforme au premier aspect de la divulgation est décrit.

La FIGURE la décrit un obturateur 400 d'écran adaptatif dans sa forme la plus simple, ledit obturateur formant une zone active pour l'écran adaptatif. Un tel obturateur 400 comprend une cellule à cristaux liquides 410 comprise entre deux substrats transparents 420. Chaque substrat comporte une électrode 430 permettant de pouvoir appliquer un champ électrique non nul à l'intérieur de la cellule à cristaux liquides 410 afin de pouvoir faire commuter l'obturateur 400 entre la configuration passante ou la configuration bloquante telles que décrites précédemment.

La FIGURE 1b décrit un obturateur 400 d'écran adaptatif, ledit obturateur 400 formant deux zones actives adjacentes pour l'écran adaptatif. Un tel obturateur 400 comprend une cellule à cristaux liquides 410 comprise entre deux substrats transparents 420. Le substrat supérieur 420 comporte deux électrodes 430 adjacentes et distantes l'une de l'autre, et le substrat inférieur 420 comprend une électrode 430 située dans une position intermédiaire entre les deux électrodes 430 du substrat supérieur. Les trois électrodes 430 permettent collectivement de pouvoir appliquer un champ électrique non nul à l'intérieur de la cellule à cristaux liquides 410 et pour chacune des zones actives, permettant ainsi de pouvoir faire commuter l'obturateur 400 entre la configuration passante ou la configuration bloquante telles que décrites précédemment.

En référence aux FIGURES 2a et 2b, une paire de lunettes 150 formant un équipement de protection optique 10 comprend un premier verre 151 et un deuxième verre 152 reliés entre eux par une monture 153.

Chaque verre 151, 152 de la paire de lunettes 150 comprend un écran adaptatif 100. Chaque écran adaptatif s'étend avantageusement sur toute la surface de chaque verre 151, 152 correspondants.

Chaque écran adaptatif 100 comprend une pluralité de zones actives, et plus particulièrement trois zones actives :
- une première zone active 121 est située sur une partie supérieure d'un verre 151, 152 ;
- une deuxième zone active 122 est située sur une partie intermédiaire d'un verre 151, 152 ;
- une troisième zone active 123 est située sur une zone inférieure d'un verre 151, 152.

Chaque zone active 121-123 comprend au moins un obturateur tel que décrits précédemment, préférentiellement du type de cristaux liquides.

En particulier, l'au moins un obturateur de chaque zone active 121-123 est agencé pour pouvoir être activé entre la configuration passante et la configuration bloquante par l'intermédiaire d'un champ électrique appliqué entre deux électrodes encadrant ledit au moins un obturateur.

Selon un premier mode de réalisation, l'au moins un obturateur prend la forme de cristaux liquides associés à au moins un polariseur optique, l'activation dudit au moins un obturateur prend alors la forme d'une polarisation optique.

Selon un deuxième mode de réalisation, l'au moins un obturateur comprend des cristaux liquides et au moins un dopant dichroïque et/ou chiral, l'activation dudit obturateur prend alors la forme d'une absorption ou d'une réflexion optique.

Afin de polariser électriquement l'au moins un obturateur de chaque zone active 121-123, chaque zone active 121-123 comprend au moins une interface de connexion électrique 131-133 permettant de relier électriquement des fils électriques conducteurs acheminant un signal de commande depuis une unité de contrôle non représentée aux électrodes.

Les interfaces de connexion électrique 131-133 sont avantageusement située en périphérie d'au moins une partie de chaque zone active 121-123 correspondante, et préférentiellement encore en périphérie de chaque écran adaptatif 100.

Plus particulièrement, dans l'exemple de réalisation illustré sur la FIGURE 2a :
- la première zone active 121 comprend une seule interface de connexion électrique 131 située sur une partie du bord supérieur de ladite première zone active 121. La deuxième zone active 122 comprend deux interfaces de connexion électrique 132 situées sur des bords latéraux opposés de ladite deuxième zone active 122. La troisième zone active 123 comprend une seule interface de connexion électrique 133 située sur un bord inférieur de ladite troisième zone active 123 ;
- la première zone active 121 prend la forme d'un bandeau de largeur constante situé sur la partie supérieure de l'écran adaptatif 100 et du verre de lunette 151, 152, de manière à former une surface perçue par le porteur de ladite paire de lunette 150 comme couvrant le ciel lorsqu'il regarde droit devant lui. En d'autres termes, la première zone active 121 couvre une surface qui correspond à la partie du champ visuel d'un conducteur située sensiblement au-dessus de la ligne d'horizon lorsque ce dernier regarde la route devant lui. La première zone active 121 correspond à la zone de pare-soleil décrite précédemment. La première zone active 121 couvre une superficie comprise entre 10% et 30% de la surface totale de l'écran adaptatif 100 ;
- la troisième zone active 123 correspond à la zone de vision de près décrite précédemment. De manière générale, elle est formée par les zones de chaque verre 151, 152 situées latéralement du côté de la monture 153 et sur une zone inférieure de l'écran adaptatif 100. Pour un conducteur de véhicule automobile portant une telle paire de lunettes 150, la troisième zone active 123 correspond à une surface qui couvre la partie du champ visuel du conducteur située sensiblement au niveau du tableau de bord dudit véhicule automobile ;
- la deuxième zone active 122 correspond à la zone active principale décrite précédemment. De manière générale, elle est située dans une position intermédiaire entre la première zone 121 et la troisième zone 123. Elle couvre une surface qui est au moins supérieure à la moitié de l'écran adaptatif 100, et préférentiellement comprise entre la 50% et 60% de la surface totale de l'écran adaptatif 100. Pour un conducteur de véhicule automobile portant une telle paire de lunettes 150, la deuxième zone active 122 correspond à une surface qui couvre la partie du champ visuel du conducteur située sensiblement au niveau de la scène de route située devant ledit véhicule automobile, comprenant notamment la route sur laquelle se trouve le véhicule automobile.

Chaque zone active 121-123 est séparée de la zone active directement adjacente par une zone inactive 110 de largeur inférieure ou égale à 2µm afin de ne pas être perceptible par le porteur de la paire de lunette 150. La zone inactive peut éventuellement comprendre au moins un obturateur non adressé par les électrodes et/ou les interfaces de connexion électrique, ou ne pas comprendre de tels obturateur.

Les fils de connexion électrique sont préférentiellement intégrés à la monture 153 de la paire de lunettes 150.

La FIGURE 2b illustre une autre variante de réalisation de la paire de lunette 150 décrite précédemment, dans laquelle les zones actives 121-123 de chaque écran adaptatif 100 sont définies et délimitées plus simplement. Plus particulièrement :
- l'interface de connexion électrique 135 est située sur tout le pourtour périphérique de chaque verre de lunette 151, 152, de sorte que chaque zone active est adressée par une partie de l'interface électrique 135. D'une manière plus précise, chaque partie périphérique située en bord du verre de lunette 151, 152 de chaque zone active 121-123 comprend une interface de connexion 135 permettant d'activer la zone active correspondante. Chaque interface électrique 135 de chaque zone active est électriquement connectée à au moins un fils de connexion électrique afin d'acheminer un signal de commande ;
- la première zone active 121 prend la forme d'un bandeau situé sur la partie supérieure de l'écran adaptatif 100 et du verre de lunette 151, 152 et séparée de la deuxième zone active 122 par une zone inactive rectiligne 110 et préférentiellement horizontale. La première zone active 121 couvre une superficie comprise entre 10% et 30% de la surface totale de l'écran adaptatif 100 ;
- la troisième zone active 123 prend la forme d'un bandeau situé sur la partie inférieure de l'écran adaptatif 100 et du verre de lunette 151, 152 et séparée de la deuxième zone active 122 par une zone inactive rectiligne 110 et préférentiellement horizontale. La troisième zone active 123 couvre une superficie comprise entre 10% et 30% de la surface totale de l'écran adaptatif 100 ;
- la deuxième zone active 122 est située dans une position intermédiaire entre la première zone active 121 et la troisième zone active 123. Elle couvre une surface qui est au moins supérieure à la moitié de l'écran adaptatif 100, et préférentiellement comprise entre la 50% et 60% de la surface totale de l'écran adaptatif 100. Elle est séparée de la troisième zone active 123 et de la première zone active 121 par une zone inactive 110 rectiligne et préférentiellement horizontale.

Chaque zone active 121-123 est séparée de la zone active 121-123 directement adjacente par une zone inactive 110 de largeur inférieure ou égale à 2µm afin de ne pas être perceptible par le porteur de la paire de lunette 150. La zone inactive peut éventuellement comprendre au moins un obturateur non adressé par les électrodes et/ou les interfaces de connexion électrique, ou ne pas comprendre de tels obturateurs.

Les fils de connexion électrique sont préférentiellement intégrés à la monture 153 de la paire de lunettes 150.

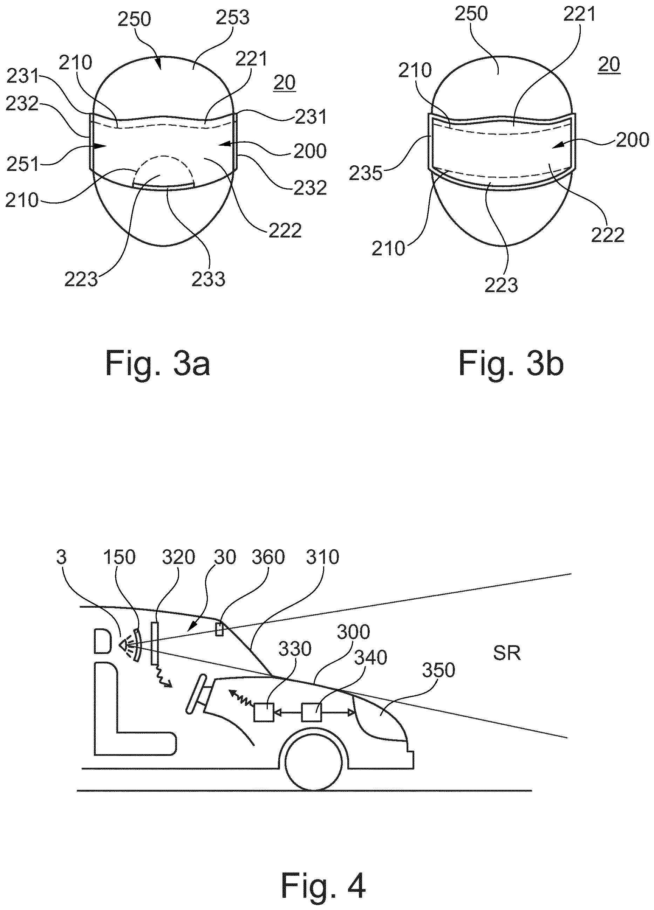

Les FIGURES 3a et 3b illustrent un casque de moto 250 formant un équipement de protection optique 20 comprend une visière 251 intégrée de manière mobile dans une coque 253.

La visière 251 comprend un écran adaptatif 200 qui s'étend avantageusement sur toute la surface de la visière 251. L'écran adaptatif 200 comprend une pluralité de zones actives 221-223, et plus particulièrement trois zones actives :
- une première zone active 221 est située sur une partie supérieure de la visière 251 ;
- une deuxième zone active 222 est située sur une partie intermédiaire de la visière 251 ;
- une troisième zone active 223 est située sur une zone inférieure de la visière 251.

Chaque zone active 221-223 comprend au moins un obturateur tel que décrit précédemment, préférentiellement du type de cristaux liquides.

En particulier, l'au moins un obturateur de chaque zone active 221-223 sont agencés pour pouvoir être activés entre la configuration passante et la configuration bloquante par l'intermédiaire d'un champ électrique appliqué entre deux électrodes encadrant lesdits obturateurs.

Selon un premier mode de réalisation, l'au moins un obturateur prend la forme de cristaux liquides associés à au moins un polariseur optique, l'activation dudit au moins un obturateur prend alors la forme d'une polarisation optique.

Selon un deuxième mode de réalisation, l'au moins un obturateur comprend des cristaux liquides et au moins un dopant dichroïque et/ou chiral, l'activation dudit obturateur prend alors la forme d'une absorption et/ou d'une réflexion optique.

Afin de polariser électriquement l'au moins un obturateur de chaque zone active 221-223, chaque zone active 221-223 comprend au moins une interface de connexion électrique 231-233 permettant de relier électriquement des fils électriques conducteurs acheminant un signal de commande depuis une unité de contrôle non représentée aux électrodes.

Les interfaces de connexion électrique 231-233 sont avantageusement située en périphérie d'au moins une partie de chaque zone active 221-223 correspondante, et préférentiellement encore en périphérie de chaque écran adaptatif 200.

Plus particulièrement, dans l'exemple de réalisation illustré sur la FIGURE 3a :
- la première zone active 221 comprend deux interfaces de connexion électrique 231 situées sur une partie de chaque bord latéral de ladite première zone active 221. La deuxième zone active 222 comprend deux interfaces de connexion électrique 232 situées sur des bords latéraux opposés de ladite deuxième zone active 222. La troisième zone active 223 comprend une seule interface de connexion électrique 233 située sur un bord inférieur de ladite troisième zone active 223 ;
- la première zone active 221 prend la forme d'un bandeau de largeur constante situé sur la partie supérieure de l'écran adaptatif 200 et de la visière 251, de manière à former une surface perçue par le porteur dudit casque de moto 250 comme couvrant le ciel lorsqu'il regarde droit devant lui. En d'autres termes, la première zone active 221 couvre une surface qui correspond à la partie du champ visuel d'un conducteur de moto située sensiblement au-dessus de la ligne d'horizon lorsque ce dernier regarde la route devant lui. La première zone active 221 correspond à la zone de pare-soleil décrite précédemment. La première zone active 221 couvre une superficie comprise entre 10% et 30% de la surface totale de l'écran adaptatif 200 ;
- la troisième zone active 223 correspond à la zone de vision de près décrite précédemment. De manière générale, elle est formée par une forme sensiblement du type d'un demi-disque dont la base est formée par le bord inférieur de la visière 251. Pour un conducteur de moto portant un tel casque de moto 250, la troisième zone active 223 correspond à une surface qui couvre la partie du champ visuel du conducteur située sensiblement au niveau du tableau de bord de ladite moto ;
- la deuxième zone active 222 correspond à la zone active principale décrite précédemment. De manière générale, elle est située dans une position intermédiaire entre la première zone 221 et la troisième zone 223. Elle couvre une surface qui est au moins supérieure à la moitié de l'écran adaptatif 200, et préférentiellement comprise entre la 50% et 60% de la surface totale de l'écran adaptatif 200. Pour un conducteur de moto portant un tel casque de moto 250, la deuxième zone active 222 correspond à une surface qui couvre la partie du champ visuel du conducteur de moto située sensiblement au niveau de la scène de route située devant ladite moto, comprenant notamment la route sur laquelle se trouve la moto.

Chaque zone active 221-223 est séparée de la zone active directement adjacente par une zone inactive 210 de largeur inférieure ou égale à 2µm afin de ne pas être perceptible par le porteur du casque de moto 250. La zone inactive 210 peut éventuellement comprendre des obturateurs non adressés par les électrodes et/ou les interfaces de connexion électrique, ou ne pas comprendre de tels obturateurs.

Les fils de connexion électrique sont préférentiellement intégrés au casque de moto 250, préférentiellement au niveau de la coque 253.

La FIGURE 3b illustre une autre variante de réalisation du casque de moto 250 décrit précédemment, dans laquelle les zones actives 221-223 de l'écran adaptatif 200 formant la visière 251 sont définies et délimitées plus simplement. Plus particulièrement :
- l'interface de connexion électrique 235 est située sur tout le pourtour périphérique de la visière 251, de sorte que chaque zone active 221-223 est adressée par une partie de l'interface électrique 235. D'une manière plus précise, chaque partie périphérique située en bordure de la visière 251 de chaque zone active 221-223 comprend une interface de connexion électrique 235 permettant de polariser la zone active correspondante. Chaque interface de connexion électrique 235 de chaque zone active 221-223 est électriquement connectée à au moins un fils de connexion électrique afin d'acheminer un signal de commande ;
- la première zone active 221 prend la forme d'un bandeau situé sur la partie supérieure de l'écran adaptatif 200 et de la visière 251. La première zone active 221 est séparée de la deuxième zone active 222 par une zone inactive rectiligne 210 et préférentiellement horizontale. La première zone active 221 couvre une superficie comprise entre 10% et 30% de la surface totale de l'écran adaptatif 200 ;
- la troisième zone active 223 prend la forme d'un bandeau situé sur la partie inférieure de l'écran adaptatif 200 de la visière 251. La troisième zone active 223 est séparée de la deuxième zone active 222 par une zone inactive rectiligne 210 et préférentiellement horizontale. La troisième zone active 223 couvre une superficie comprise entre 10% et 30% de la surface totale de l'écran adaptatif 200 ;
- la deuxième zone active 222 est située dans une position intermédiaire entre la première zone active 221 et la troisième zone active 223. Elle couvre une surface qui est au moins supérieure à la moitié de l'écran adaptatif 200, et préférentiellement comprise entre la 50% et 60% de la surface totale de l'écran adaptatif 200. Elle est séparée de la troisième zone active 223 et de la première zone active 221 par une zone inactive 210 rectiligne et préférentiellement horizontale.

Chaque zone active 221-223 est séparée de la zone active 221-223 directement adjacente par une zone inactive 210 de largeur inférieure ou égale à 2µm afin de ne pas être perceptible par le porteur du casque de moto 250. La zone inactive 210 peut éventuellement comprendre des obturateurs non adressés par les électrodes et/ou les interfaces de connexion électrique, ou ne pas comprendre de tels obturateurs.

Les fils de connexion électrique sont préférentiellement intégrés à la coque 253 du casque de moto 250.

La FIGURE 4 illustre une vue schématique en coupe partielle d'un système d'aide à la conduite 30 conforme au troisième aspect de la divulgation.

Un véhicule automobile 300 est équipé de manière conventionnelle par un dispositif d'éclairage 350 agencé pour émettre un faisceau d'éclairage d'une scène de route SR grâce à au moins une source lumineuse. Le véhicule 300 est piloté par un conducteur, symbolisé par son œil 3. La scène de route SR correspond à ce qu'observe le conducteur 3 du véhicule 300. De manière schématique, le conducteur 3 observe la scène de route SR située devant le véhicule 300 et à travers le pare-brise 310.

Un écran adaptatif est disposé dans le champ de vision du conducteur 3, entre ce-dernier et la scène de route SR. Selon différents modes de divulgation, l'écran adaptatif peut être constitué :
- d'un écran 320 placé préférentiellement entre le conducteur 3 et le pare-brise 310. De manière avantageuse, l'écran 320 est rabattable à la façon d'un pare-soleil ou enroulable ;
- du pare-brise 310 lui-même ; ou
- d'une paire de lunettes 150 portée par le conducteur 3, à l'instar de lunettes de soleil ou de lunettes correctrices.

Par commodité, ces trois modes de réalisation ont été représentés simultanément sur la FIGURE 4. Ce ne sont cependant que des variantes de réalisation, chacun d'entre eux tendant à obtenir le même résultat.

Dans la suite de la description, le terme « écran adaptatif » sera utilisé pour désigner indifféremment l'un de ces trois modes de réalisation.

Pour chacun des modes de réalisation, l'écran adaptatif comprend une pluralité de zones actives, chaque zone active étant formée par au moins un obturateur agencés pour obturer la zone active correspondante à au moins une fréquence d'oscillation donnée, tel qu'il a été décrit précédemment. Chaque zone active de l'écran adaptatif peut prendre deux configurations consécutives et par alternance :
- une première configuration, dite passante, pour laquelle les obturateurs sont agencés pour laisser passer un rayon lumineux au travers de ladite zone active de l'écran adaptatif ;
- une seconde configuration, dite bloquante, pour laquelle les obturateurs sont agencés pour ne pas laisser passer un rayon lumineux au travers de ladite zone active de l'écran adaptatif.

Selon un premier mode de réalisation, le cas l'au moins un obturateur prend la forme de cristaux liquides associés à au moins un polariseur optique, l'activation desdits dudit au moins un obturateur prend alors la forme d'une polarisation optique.

Selon un deuxième mode de réalisation, l'au moins un obturateur comprend des cristaux liquides et au moins un dopant dichroïque et/ou chiral, l'activation desdits dudit obturateurs oscillant prend alors la forme d'une absorption et/ou d'une réflexion optique.

De manière avantageuse, chaque zone active de l'écran adaptatif est pilotée par un signal de commande qui fait commuter les obturateurs correspondant entre la première et la seconde configuration. Le signal de commande est caractérisé par au moins trois paramètres qui permettent de configurer une transmittance moyenne de la zone active correspondante :
- une fréquence de commutation ; et/ou
- une phase de commutation; et/ou
- un rapport cyclique de commutation, avantageusement invariant, par exemple à une valeur de 50%.

Conformément au troisième aspect, les paramètres de chaque signal de commande pilotant une zone active peuvent être contrôlés et modifiés en fonction d'au moins une mesure réalisée sur la scène de route SR par un photodétecteur 360.

Le photodétecteur 360 est avantageusement situé entre l'écran adaptatif et le conducteur 3. Dans le cas où l'écran adaptatif est situé sur le pare-brise 310, alors le photodétecteur 360 est situé derrière ledit pare-brise 310, à l'intérieur du véhicule 30. Dans le cas où l'écran adaptatif est situé sur l'écran 320, alors le photodétecteur 360 est situé derrière ledit écran 320, entre l'écran 320 et le conducteur 3. Dans le cas où l'écran adaptatif est situé sur la paire de lunettes 150, alors le photodétecteur 360 est situé derrière ladite paire de lunettes 150, entre la paire de lunettes 150 et le conducteur 3. Éventuellement, le photodétecteur 360 peut aussi être situé devant la paire de lunette 150.

Le photodétecteur 360 est agencé pour mesurer au moins un signal dit incident pouvant provenir d'une source lumineuse située sur la scène de route, de l'autre côté de l'écran par rapport au conducteur 3. À titre d'exemple non limitatif, le photodétecteur 360 peut avantageusement comprendre une caméra vidéo ou une photodiode. Préférentiellement, le photodétecteur est agencé pour pouvoir détecter au moins un rayon lumineux dont la fréquence est comprise dans le spectre visible.

Le dispositif d'aide à la conduite comprend avantageusement une unité de contrôle 330 agencée pour contrôler chaque zone active de l'écran adaptatif. L'unité de contrôle 330peut avantageusement prendre la forme d'une centrale de commande du véhicule 30.

De manière préférentielle, l'unité de contrôle 330 est reliée à une autre contrôle 340 qui commande l'alimentation du dispositif d'éclairage 350 du véhicule 30. Le dispositif d'éclairage 350 comprend au moins une source lumineuse agencée pour émette un faisceau lumineux sur la scène de route SR. De manière préférentielle, le faisceau lumineux est d'intensité périodiquement variable entre une valeur maximale et une valeur minimale. En d'autres termes, le dispositif d'éclairage 350 est configuré pour émettre un faisceau lumineux pulsé, dont l'intensité lumineuse et/ou la fréquence de pulsation et/ou la phase de pulsation sont contrôlées par un signal de commande généré par ladite autre unité de contrôle 340 du dispositif d'éclairage 350.

Avantageusement l'unité de contrôle 340 du dispositif d'éclairage 350 est agencée et configurée pour pouvoir communiquer avec l'unité de contrôle 330 du système d'aide à la conduite afin de pouvoir au moins transmettre au moins un paramètre du signal de commande contrôlant le dispositif d'éclairage 350.

Lorsque l'écran adaptatif est par exemple mobile ou éloigné de contrôle 330, notamment dans le cas de l'utilisation de la paire de lunettes 150 ou de l'écran 320, le dispositif d'aide à la conduite peut comprendre un dispositif de communication filaires ou sans fils, par exemple en utilisant un protocole de communication sans-fil déterminé, comme par exemple selon les normes IEEE 802.15.1 ainsi que toutes ses extensions communément dénommées par la marque déposée Bluetooth ou IEEE 802.11 communément dénommé par la marque déposée Wifi, et plus spécifiquement la norme IEEE 802.11p concernant le wifi appliqué dans le domaine automobile.

Avantageusement, le dispositif de communication comprend aussi un émetteur-receveur d'ondes connectée à contrôle 330 et configuré pour au moins envoyer vers l'écran adaptatif au moins un signal de commande en fonction des données mesurées par le photodétecteur et/ou des paramètres de fonctionnement du dispositif d'éclairage.

En synthèse, la divulgation concerne un écran adaptatif comprenant au moins un obturateur à cristaux liquides, au moins un des obturateurs comprenant au moins deux zones actives adressées par un signal de commande permettant de faire commuter l'au moins un obturateur correspondant entre une configuration passante dans laquelle une transmittance est égale à une valeur maximale, et une configuration bloquante dans laquelle la transmittance est égale à une valeur minimale, caractérisé en ce qu'une zone active dite principale couvre une surface de l'écran adaptatif inférieure ou égale à 60% de la surface dudit écran adaptatif afin de réduire le temps de réponse de ladite zone active principale

La divulgation concerne aussi différents dispositifs mettant en œuvre un tel écran adaptatif, tel que par exemple des équipements de protection optique, un système d'aide à la conduite et un véhicule intégrant un tel système d'aide à la conduite.

Bien sûr, la divulgation n'est pas limitée aux exemples qui viennent d'être décrits et de nombreux aménagements peuvent être apportés à ces exemples sans sortir du cadre de la divulgation Notamment, les différentes caractéristiques, formes, variantes et modes de réalisation peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

## Revendications

1. Équipement de protection optique (10, 20) comprenant un écran adaptatif (100, 200) et un support (153, 253) agencé pour maintenir l'écran adaptatif (100, 200) devant les yeux d'un individu portant ledit équipement de protection optique (10, 20), l'écran adaptatif (100, 200) comprenant au moins un obturateur (400) à cristaux liquides, au moins un des obturateurs (400) comprenant au moins deux zones actives (121-123, 221-223) adaptées à être adressées par un signal de commande permettant de faire commuter l'au moins un obturateur (400) correspondant entre une configuration passante dans laquelle une transmittance est égale à une valeur maximale, et une configuration bloquante dans laquelle la transmittance est égale à une valeur minimale, et dans lequel une des au moins deux zones actives, (121-123, 221-223) dite zone active principale couvre une surface de l'écran adaptatif (100, 200) inférieure ou égale à 60% de la surface dudit écran adaptatif (100, 200), et est située dans une zone centrale de l'écran adaptatif (100, 200) et **caractérisé en ce que** ladite zone active principale s'étend latéralement d'un bord à l'autre de l'écran adaptatif (100, 200), et est définie par deux électrodes (430) situées de part et d'autre de l'obturateur à cristaux liquides.

2. Equipement de protection optique (10, 20) selon la revendication précédente **caractérisé en ce que** l'écran adaptatif (100, 200) comprend au moins trois électrodes (430) dont deux sont situées d'un premier côté de l'au moins un obturateur (400) et une troisième est située d'un deuxième côté de l'au moins un obturateur (400), les électrodes (430) permettant d'appliquer un champ électrique non nul entre elles pour configurer ledit au moins un obturateur (400) de chaque zone active (121-123, 221-223) dans la configuration passante ou bloquante, chaque zone active (121-123, 221-223) pouvant être configurée indépendamment des autres zones actives (121-123, 221-223).

3. Equipement de protection optique (10, 20) selon l'une quelconque des revendications précédentes, **caractérisé en ce que** l'au moins un obturateur (400) est adapté pour être configuré en fonction d'au moins un paramètre du signal de commande, le paramètre étant choisi parmi une fréquence de commutation et/ou une phase de commutation et/ou un rapport cyclique de commutation.

4. Equipement de protection optique (10, 20) selon la revendication précédente, **caractérisé en ce qu'**au moins un des obturateurs est adapté à disposer d'une fréquence de commutation de la zone active (121-123, 221-223) principale supérieure ou égale à 200 Hz.

5. Equipement de protection optique (10, 20) selon l'une quelconque des revendications précédentes, **caractérisé en ce que** l'écran adaptatif (100, 200) comprend des conducteurs électriques connectés électriquement à chacune des électrodes (430) au niveau d'au moins une interface de connexion électrique (131-133, 135, 231-233, 235), chaque interface de connexion électrique (131-133, 135, 231-233, 235) s'étendant le long d'une zone active (121-123, 221-223).

6. Équipement de protection optique (10, 20) selon l'une des revendications précédentes, **caractérisé en ce que** l'au moins un obturateur (400) comprend un mélange à cristal liquide et au moins un polariseur.

7. Équipement de protection optique (10, 20) selon l'une des revendications 1 à 5, **caractérisé en ce que** l'au moins un obturateur (400) comprend un mélange à cristal liquide avec au moins un dopant dichroïque et/ou chiral.

8. Équipement de protection optique (10, 20) selon l'une quelconque des revendications précédentes, **caractérisé en ce qu'**il comprend une unité de contrôle configurée pour générer le signal de commande de manière à faire commuter l'au moins un obturateur (400) d'au moins une zone active (121-123, 221-223) entre la configuration passante et la configuration bloquée.

9. Système d'aide à la conduite (30) d'un véhicule automobile (300) comprenant :
- un équipement de protection optique (10, 20) selon l'une quelconque des revendications 1 à 8 ;
- une unité de contrôle (330) d'au moins une partie des zones actives (121-123, 221-223) de l'écran adaptatif (100, 200), l'unité de contrôle (330) étant configurée pour générer au moins un signal de commande de l'au moins un obturateur (400) de l'écran adaptatif (100, 200afin de faire commuter l'au moins un obturateur (400) de la zone active (121-123, 221-223) correspondante entre la configuration passante et la configuration bloquée, chaque zone active (121-123, 221-223) étant contrôlée par un signal de commande égal ou différent du signal de commande des autres zones actives (121-123, 221-223).

10. Système d'aide à la conduite (30) selon la revendication précédente, **caractérisé en ce qu'**il comprend en outre :
- un photodétecteur (360) agencé pour détecter au moins un rayon lumineux atteignant le véhicule automobile (300) depuis une scène de route (SR) située devant ledit véhicule automobile (300) ; et
- une unité de traitement configurée pour déterminer une intensité lumineuse du rayon lumineux, l'au moins un signal de commande généré par l'unité de contrôle (330) étant déterminé en fonction de l'intensité lumineuse.

11. Système d'aide à la conduite (30) selon la revendication 10, **caractérisé en ce qu'**au moins une zone active (121-123, 221-223) de l'écran adaptatif (100, 200) est configurée pour réduire un scintillement et/ou un éblouissement détecté par le photodétecteur (360).

12. Système d'aide à la conduite (30) selon l'une quelconque des revendications 9 ou 11, **caractérisé en ce que** l'écran adaptatif (100, 200) est formé par :
- au moins un verre de lunettes (150) portées par le conducteur (3) du véhicule automobile (300) ; ou
- au moins une partie d'une visière (251) d'un casque (250) porté par le conducteur (3) du véhicule automobile (300).

13. Système d'aide à la conduite (30) selon la revendication précédente, dans lequel l'écran adaptatif (100, 200) est formé par les lunettes (150) ou le casque (250), **caractérisé en ce que** l'écran adaptatif (100, 200) comprend :
- une zone active (121, 221) dite de pare-soleil et située sur une partie supérieure de l'écran adaptatif (100, 200) et s'étendant latéralement d'un bord à l'autre de l'écran adaptatif (100, 200) ; et/ou
- une zone active (123, 223) dite de vision de près située dans un coin inférieur de l'écran adaptatif (100, 200), la surface de ladite zone de vision de près (123, 223) étant supérieure ou égale au quart de la surface de l'écran adaptatif (100, 200).

14. Système d'aide à la conduite (30) selon l'une quelconque des revendications 9 à 13, **caractérisé en ce qu'**il comprend un oculomètre agencé pour mesurer un mouvement des yeux du conducteur (3) du véhicule automobile (300) et/ou un dispositif de suivi d'un mouvement de la tête du conducteur (3) du véhicule automobile (300), au moins une zone active (121-123, 221-223) de l'écran adaptatif (100, 200) étant configurée en fonction dudit mouvement des yeux et/ou dudit mouvement de la tête respectivement.

## Patentansprüche

1. Optische Schutzeinrichtung (10, 20) mit einem adaptiven Bildschirm (100, 200) und einem Halter (153, 253), der angeordnet ist, um den adaptiven Bildschirm (100, 200) vor den Augen einer Person zu halten, die die optische Schutzeinrichtung (10, 20) trägt, wobei der adaptive Bildschirm (100, 200) mindestens einen Flüssigkristallverschluss (400) umfasst, mindestens einer der Verschlüsse (400) mit mindestens zwei aktiven Zonen (121-123, 221-223), die durch ein Steuersignal adressierbar sind, das es ermöglicht, den mindestens einen entsprechenden Verschluss (400) zwischen einer Durchgangskonfiguration umzuschalten, in der eine Durchlässigkeit gleich einem Maximalwert ist, und eine Blockierkonfiguration, bei der die Durchlässigkeit gleich einem Minimalwert ist, und bei der eine der mindestens zwei aktiven Zonen (121-123, 221-223) die als aktive Hauptzone genannt ist, eine Oberfläche des adaptiven Bildschirms (100, 200) bedeckt, die kleiner oder gleich 60% der Oberfläche des adaptiven Bildschirms (100, 200) ist, in einem zentralen Bereich des adaptiven Bildschirms (100, 200) angeordnet ist und **dadurch gekennzeichnet ist, dass** sich der aktive Hauptzone seitlich von einer Kante zur anderen des adaptiven Bildschirms (100, 200) erstreckt und durch zwei Elektroden (430) definiert ist, die sich auf beiden Seiten des Flüssigkristallverschlusses befinden.

2. Optische Schutzeinrichtung (10, 20) nach dem vorhergehenden Anspruch, **dadurch gekennzeichnet, dass** der adaptive Bildschirm (100, 200) mindestens drei Elektroden (430) umfasst, von denen zwei auf einer ersten Seite des mindestens einen Verschlusses (400) und ein dritter auf einer zweiten Seite des mindestens einen Verschlusses (400) angeordnet sind, die Elektroden (430), die es ermöglichen, ein elektrisches Feld ungleich Null zwischen ihnen anzulegen, um den mindestens einen Verschluss (400) jeder aktiven Zone (121-123, 221-223) in der Durchgangs- oder Blockierkonfiguration zu konfigurieren, wobei jede aktive Zone (121-123, 221-223) unabhängig von den anderen aktiven Zonen (121-123, 221-223) konfigurierbar ist.

3. Optische Schutzeinrichtung (10, 20) nach einem der vorstehenden Ansprüche, **dadurch gekennzeichnet, dass** die mindestens einen Verschluss (400) entsprechend mindestens einem Parameter des Steuersignals konfigurierbar ist, wobei der Parameter aus einer Schaltfrequenz und/oder einer Schaltphase und/oder einem Schaltverhältnis ausgewählt ist.

4. Optische Schutzeinrichtung (10, 20) nach dem vorstehenden Anspruch, **dadurch gekennzeichnet, dass** mindestens einer der Verschlüsse so ausgelegt ist, dass er eine Schaltfrequenz der aktiven Hauptzone (121-123, 221-223) größer oder gleich 200 Hz aufweist.

5. Optische Schutzeinrichtung (10, 20) nach einem der vorstehenden Ansprüche, **dadurch gekennzeichnet, dass** der adaptive Bildschirm (100, 200) elektrische Leiter umfasst, die elektrisch mit jeder der Elektroden (430) an mindestens einer elektrischen Verbindungsschnittstelle (131-133, 135, 231-233, 235) verbunden sind, wobei sich jede elektrische Verbindungsschnittstelle (131-133, 135, 231-233, 235) entlang einer aktiven Zone (121-123, 221-223) erstreckt.

6. Optische Schutzeinrichtung (10, 20) nach einem der vorstehenden Ansprüche, **dadurch gekennzeichnet, dass** der mindestens eine Verschluss (400) eine Flüssigkristallmischung und mindestens einen Polarisator umfasst.

7. Optische Schutzeinrichtung (10, 20) nach einem der Ansprüche 1 bis 5, **dadurch gekennzeichnet, dass** der mindestens eine Verschluss (400) eine Flüssigkristallmischung mit mindestens einem dichroitischen und/oder chiralen Dotierstoff umfasst.

8. Optische Schutzeinrichtung (10, 20) nach einem der vorstehenden Ansprüche, **dadurch gekennzeichnet, dass** sie eine Steuereinheit umfasst, die konfiguriert ist, um das Steuersignal so zu erzeugen, dass sie den mindestens einen Verschluss (400) mindestens einer aktiven Zone (121-123, 221-223) zwischen der Durchgangskonfiguration und der Blockierkonfiguration umschaltet.

9. Fahrassistenzsystem (30) für ein Kraftfahrzeug (300), umfassend:
- eine optische Schutzeinrichtung (10, 20) nach einem der Ansprüche 1 bis 8;
- eine Steuereinheit (330) für mindestens einen Abschnitt der aktiven Zone (121-123, 221-223) des adaptiven Bildschirms (100, 200), wobei die Steuereinheit (330) konfiguriert ist, um mindestens ein Steuersignal für den mindestens einen Verschluss (400) des adaptiven Bildschirms (100, 200) um den mindestens einen Verschluss (400) der entsprechenden aktiven Zone (121-123, 221-223) zwischen der Durchgangskonfiguration und der Blockierkonfiguration zu schalten, wobei jede aktive Zone (121-123, 221-223) durch ein Steuersignal gesteuert wird, das gleich oder verschieden vom Steuersignal der anderen aktiven Zonen (121-123, 221-223) ist.

10. Fahrassistenzsystem (30) nach dem vorstehenden Anspruch, **dadurch gekennzeichnet, dass** es ferner umfasst:
- einen Photodetektor (360), der angeordnet ist, um mindestens einen Lichtstrahl zu erfassen, der das Kraftfahrzeug (300) von einer Straßenszene (SR) erreicht, die sich vor dem Kraftfahrzeug (300) befindet; und
- eine Verarbeitungseinheit, die konfiguriert ist, um eine Lichtintensität des Lichtstrahls zu bestimmen, wobei das mindestens eine von der Steuereinheit (330) erzeugte Steuersignal als Funktion der Lichtintensität bestimmt wird.

11. Fahrassistenzsystem (30) nach Anspruch 10, **dadurch gekennzeichnet, dass** mindestens eine aktive Zone (121-123, 221-223) des adaptiven Bildschirms (100, 200) konfiguriert ist, um das vom Photodetektor (360) erfasste Flackern und/oder Blenden zu reduzieren.

12. Fahrassistenzsystem (30) nach einem der Ansprüche 9 oder 11, **dadurch gekennzeichnet, dass** der adaptive Bildschirm (100, 200) gebildet ist durch :
- mindestens eine Brillenlinse (150), die vom Fahrer (3) des Kraftfahrzeugs (300) getragen wird; oder
- mindestens einen Teil eines Visiers (251) eines Helmes (250), der vom Fahrer (3) des Kraftfahrzeugs (300) getragen wird.

13. Fahrassistenzsystem (30) nach dem vorstehenden Anspruch, wobei der adaptive Bildschirm (100, 200) durch die Brille (150) oder den Helm (250) gebildet ist, **dadurch gekennzeichnet, dass** der adaptive Bildschirm (100, 200) umfasst:
- eine aktive Zone (121, 221), die als Sonnenblende bezeichnet wird und sich an einem oberen Teil des adaptiven Bildschirms (100, 200) befindet und sich seitlich von einer Kante zur anderen des adaptiven Bildschirms (100, 200) erstreckt; und/oder
- eine aktive Zone (123, 223), genannt ein Nahsichtbereich, der sich in einer unteren Ecke des adaptiven Bildschirms (100, 200) befindet, wobei der Bereich des Nahsichtbereichs (123, 223) größer oder gleich einem Viertel des Bereichs des adaptiven Bildschirms (100, 200) ist.

14. Fahrassistenzsystem (30) nach einem der Ansprüche 9 bis 13, **dadurch gekennzeichnet, dass** es ein Okulometer umfasst, das zum Messen einer Bewegung der Augen des Fahrers (3) des Kraftfahrzeugs (300) und/oder eine Vorrichtung zum Überwachen einer Bewegung des Fahrerkopfes (3) des Kraftfahrzeugs (300) angeordnet ist, wobei mindestens eine aktive Zone (121-123, 221-223) des adaptiven Bildschirms (100, 200) entsprechend der Bewegung der Augen bzw. der Bewegung des Kopfes konfiguriert ist.

## Claims

1. Optical protection equipment (10, 20) comprising an adaptive screen (100, 200) and a holder (153, 253) arranged to hold the adaptive screen (100, 200) in front of the eyes of an individual wearing said optical protection equipment (10, 20), the adaptive screen (100, 200) comprising at least one liquid crystal shutter (400), at least one of the shutters (400) comprising at least two active zones (121-123, 221-223) adapted to be addressed by a control signal enabling the at least one corresponding shutter (400) to be switched between a passing configuration in which a transmittance is equal to a maximum value, and a blocking configuration in which the transmittance is equal to a minimum value, and in which one of the at least two active zones (121-123, 221-223) said main active zone covers a surface of the adaptive screen (100, 200) less than or equal to 60% of the surface of said adaptive screen (100, 200), is located in a central area of the adaptive screen (100, 200) and **characterized in that** said main active zone extends laterally from one edge to the other of the adaptive screen (100, 200), and is defined by two electrodes (430) located on either side of the liquid crystal shutter.

2. Optical protective equipment (10, 20) according to the preceding claim, **characterized in that** the adaptive screen (100, 200) comprises at least three electrodes (430), two of which are located on a first side of the at least one shutter (400) and a third is located on a second side of the at least one shutter (400), the electrodes (430) allowing to apply a non-zero electric field between them to configure said at least one shutter (400) of each active zone (121-123, 221-223) in the passing or blocking configuration, each active zone (121-123, 221-223) being configurable independently of the other active zones (121-123, 221-223).

3. Optical protection equipment (10, 20) according to any one of the preceding claims, **characterized in that** the at least one shutter (400) is adapted to be configured according to at least one parameter of the control signal, the parameter being chosen from a switching frequency and/or a switching phase and/or a switching duty cycle.

4. Optical protective equipment (10, 20) according to the preceding claim, **characterized in that** at least one of the shutters is adapted to have a switching frequency of the main active zone (121-123, 221-223) greater than or equal to 200 Hz.

5. Optical protective equipment (10, 20) according to any one of the preceding claims, **characterized in that** the adaptive screen (100, 200) comprises electrical conductors electrically connected to each of the electrodes (430) at at least one electrical connection interface (131-133, 135, 231-233, 235), each electrical connection interface (131-133, 135, 231-233, 235) extending along an active zone (121-123, 221-223).

6. Optical protective equipment (10, 20) according to any one of the preceding claims, **characterized in that** the at least one shutter (400) comprises a liquid crystal mixture and at least one polarizer.

7. Optical protective equipment (10, 20) according to any one of claims 1 to 5, **characterized in that** the at least one shutter (400) comprises a liquid crystal mixture with at least one dichroic and/or chiral dopant.

8. Optical protection equipment (10, 20) according to any one of the preceding claims, **characterized in that** it comprises a control unit configured to generate the control signal in such a way as to switch the at least one shutter (400) of at least one active zone (121-123, 221-223) between the passing configuration and the blocked configuration.

9. Driving assistance system (30) for a motor vehicle (300) comprising:
- a optical protective equipment (10, 20) according to any of claims 1 to 8;
- a control unit (330) for at least a portion of the active zones (121-123, 221-223) of the adaptive screen (100, 200), the control unit (330) being configured to generate at least one control signal for the at least one shutter (400) of the adaptive screen (100, 200) in order to switch the at least one shutter (400) of the corresponding active zone (121-123, 221-223) between the passing configuration and the blocked configuration, each active zone (121-123, 221-223) being controlled by a control signal equal to or different from the control signal of the other active zones (121-123, 221-223).

10. Driving assistance system (30) according to the preceding claim, **characterized in that** it further comprises:
- a photodetector (360) arranged to detect at least one light beam reaching the motor vehicle (300) from a road scene (SR) located in front of said motor vehicle (300); and
- a processing unit configured to determine a light intensity of the light beam, the at least one control signal generated by the control unit (330) being determined as a function of the light intensity.

11. Driving assistance system (30) according to claim 10, **characterized in that** at least one active zone (121-123, 221-223) of the adaptive screen (100, 200) is configured to reduce flicker and/or glare detected by the photodetector (360).

12. Driving assistance system (30) according to any one of claims 9 or 11, **characterized in that** the adaptive screen (100, 200) is formed by :
- at least one spectacle lens (150) worn by the driver (3) of the motor vehicle (300); or
- at least a part of a visor (251) of a helmet (250) worn by the driver (3) of the motor vehicle (300).

13. Driving assistance system (30) according to the preceding claim, wherein the adaptive screen (100, 200) is formed by the glasses (150) or helmet (250), **characterized in that** the adaptive screen (100, 200) comprises :
- an active zone (121, 221) called a sun visor and located on an upper part of the adaptive screen (100, 200) and extending laterally from one edge to the other of the adaptive screen (100, 200); and/or
- an active zone (123, 223) called a near vision zone located in a lower corner of the adaptive screen (100, 200), the area of said near vision zone (123, 223) being greater than or equal to a quarter of the area of the adaptive screen (100, 200).

14. Driving assistance system (30) according to any one of claims 9 to 13, **characterized in that** it comprises an oculometer arranged to measure a movement of the eyes of the driver (3) of the motor vehicle (300) and/or a device for monitoring a movement of the driver's head (3) of the motor vehicle (300), at least one active zone (121-123, 221-223) of the adaptive screen (100, 200) being configured according to said movement of the eyes and/or said movement of the head respectively.
